# EUROPEAN PATENT SPECIFICATION

(11) **EP 4 456 046 B1**
(45) Date of publication and mention of the grant of the patent: **18.06.2025**
(21) Application number: 24171697.6
(22) Date of filing: 22.04.2024
(51) Int. Cl.: G09B 23/30, G09B 23/32, G09B 23/34

(54) **CRICOTHYROTOMY TRAINER**
KRIKOTHYREOTOMIETRAINER
DISPOSITIF D'ENTRAÎNEMENT POUR CRICOTHYROTOMIE

(30) Priority: 25.04.2023 US 202363498081 P; 16.02.2024 US 202418444220
(43) Date of publication of application: 30.10.2024
(73) Proprietor: Levitan, Richard M., Orford, NH 03777 (US)
(72) Inventor: LEVITAN, Richard M., Orford, NH 03777 (US); MILLER, Andrew, Crowthorne, RG45 6DU (GB)
(74) Representative: De Vries & Metman

(56) References cited:
- WO-A1-2022/178398
- US-A1- 2014 154 656
- US-A1- 2020 219 416
- US-A1- 2022 246 062

## Description

### Cross-Reference to Related Applications

This application claims the benefit of the filing date of U.S. provisional application no. 63/498,081, filed on 4/25/23,.

### BACKGROUND

### Field of the Disclosure

The present disclosure relates to task trainers for critical medical procedures and, more specifically but not exclusively, to task trainers for cricothyrotomies.

### Description of the Related Art

This section introduces aspects that may help facilitate a better understanding of the disclosure. Accordingly, the statements of this section are to be read in this light and are not to be understood as admissions about what is prior art or what is not prior art.

Task trainers for critical medical procedures incorporate visual and tactile anatomic realism with structural components that permit operators to practice in a manner that recreates their stepwise performance in an actual patient. Most task trainers are made of heavy plastic, rubber, and latex. Trainers designed for surgical procedures typically have a latex skin section that can be replaced in small sections after use. These trainers are utilized in simulation training centers in which operators are assigned to visit for regular training, as the skills they are practicing require frequent repetition to maintain competence. This is particularly necessary for high-acuity, low-opportunity procedures such as surgical cricothyrotomy, chest tube thoracostomy, and endotracheal intubation.
US 2014/154656 is related to a Tactical Combat Casualty Care Trainer For Hyper-Realistic^{™} Emergency Medical Training ("TCCC") which includes a tactical combat training device for the purpose of providing an emergency medical services provider hands-on training. The TCCC includes an artificial human skeleton having specific anatomical features which provide the trainee or provider with appropriate tactile response analogous to a real human patient, thereby increasing the fidelity of training and improving the skills necessary to conduct procedures such as cricothyrotomy, intrasosseous infusion, CPR and other medical services to human patients. The skeleton is covered by a realistic coating that simulates human skin, which adds to the TCCC's training value. The TCCC also includes a removable trachea module and training pucks located at the sternum as well as the left and right proximal humeral heads to simulate the use of infusion-type devices.

### SUMMARY

Historically task trainers have not been designed for overall low cost and ease of shipping, made of lightweight materials, or engineered to have a very inexpensive skin analogue. A task trainer with these characteristics could be deployed in all settings wherever medical personnel work, whether in civilian settings or military settings, wherever a medical detachment is present. It would allow exponentially greater frequency of practice by all operators, rather than depending on traditional trainers used in teaching hospital medical simulation centers that require the operators to go to the training center.

In certain embodiments, the present disclosure is a trainer for cricothyrotomy. The trainer comprises a case and a model of a patient mounted within the case. The model comprises a hyoid analogue; a thyroid analogue adjacent to the hyoid analogue; a cricoid analogue adjacent to the thyroid analogue; a tracheal analogue adjacent to the cricoid analogue; and a skin analogue adapted to cover the hyoid, thyroid, cricoid, and tracheal analogues.

### BRIEF DESCRIPTION OF THE DRAWINGS

Embodiments of the disclosure will become more fully apparent from the following detailed description, the appended claims, and the accompanying drawings in which like reference numerals identify similar or identical elements.
FIG. 1A is a perspective view of the trainer 100 with the cover 112 of the case 102 in its completely closed position on the base 104.
FIG. 1B is a perspective view of the trainer 100 with the cover 112 partially removed from the base 104 and without the skin analogue 150, thereby exposing the model 122 mounted within the base 104.
FIG. 1C is a side view of the trainer 100 of FIG. 1B.
FIG. 1D is a plan view of the trainer 100 of FIG. 1B.
FIG. 1E is a perspective view of the trainer 100 with the cover 112 partially removed from the base 104 and with the skin analogue 150 applied, thereby removing from view the model 122 mounted within the base 104.
FIG. 1F is a side view of the trainer 100 of FIG. 1E.
FIG. 1G is a plan view of the trainer 100 of FIG. 1E.
FIG. 1H is an exploded perspective view of the trainer of FIG. 1E.
FIGs. 2A-2D show plan, side, proximal end, and perspective views of the base 104 of trainer 100.
FIGs. 3A-3D show plan, side, proximal end, and perspective views of the cover 112 of trainer 100.
FIGs. 4A-4D show plan, side, proximal end, and perspective views of the cricoid analogue 136 of trainer 100.
FIGs. 5A-5D show plan, side, proximal end, and perspective views of the hyoid/thyroid analogue 124 of trainer 100.
FIGs. 6A-6D show plan, side, proximal end, and perspective views of the tracheal analogue 144 of trainer 100.
FIGs. 7A-7E show plan, side, proximal end, cross-sectional, and perspective views of the skin 150 of trainer 100.

### DETAILED DESCRIPTION

Detailed illustrative embodiments of the present disclosure are disclosed herein. However, specific structural and functional details disclosed herein are merely representative for purposes of describing example embodiments of the present disclosure. The present disclosure may be embodied in many alternate forms and should not be construed as limited to only the embodiments set forth herein. Further, the terminology used herein is for the purpose of describing particular embodiments only and is not intended to be limiting of example embodiments of the disclosure.

As used herein, the singular forms "a," "an," and "the," are intended to include the plural forms as well, unless the context clearly indicates otherwise. It further will be understood that the terms "comprises," "comprising," "contains," "containing," "includes," and/or "including," specify the presence of stated features, steps, or components, but do not preclude the presence or addition of one or more other features, steps, or components. It also should be noted that in some alternative implementations, the functions/acts noted may occur out of the order noted in the figures. For example, two figures shown in succession may in fact be executed substantially concurrently or may sometimes be executed in the reverse order, depending upon the functions/acts involved.

The accompanying figures illustrate cricothyrotomy task trainers of the present disclosure that are designed to be lightweight, self-contained in its own case which opens to expand its size, and anatomically and mechanically realistic of male/female laryngeal structures, and incorporate a very inexpensive three-layer skin analogue. There are no commercial task trainers for cricothyrotomy made with female laryngeal structures or with three-layer skin analogues.

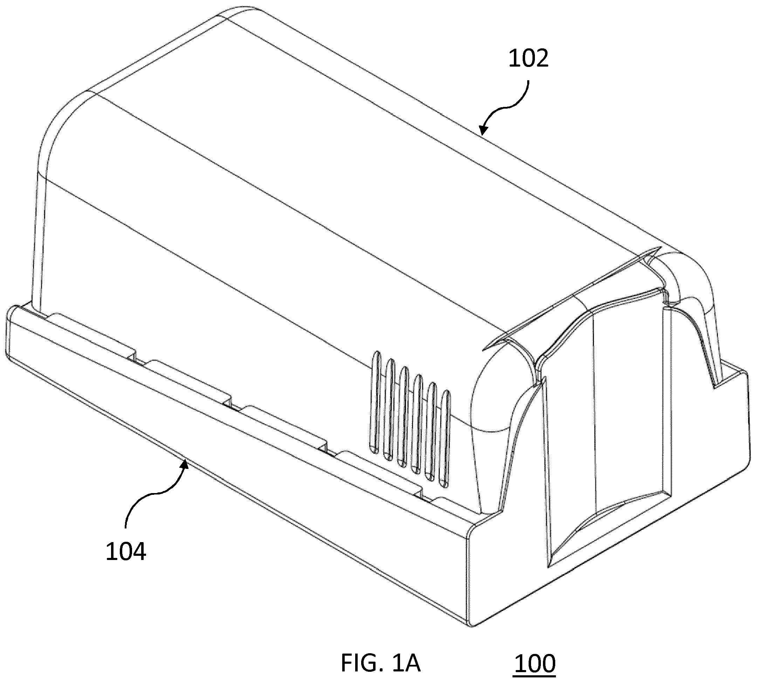

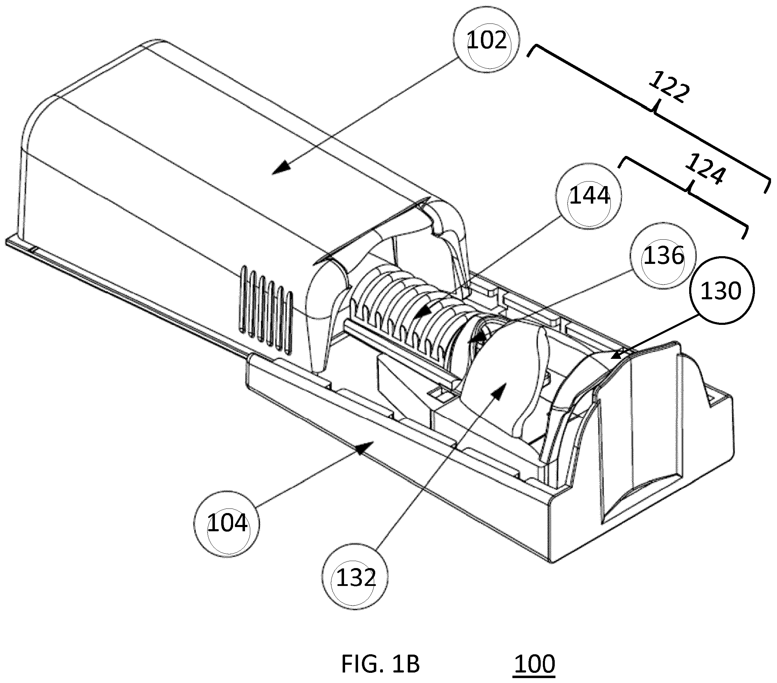

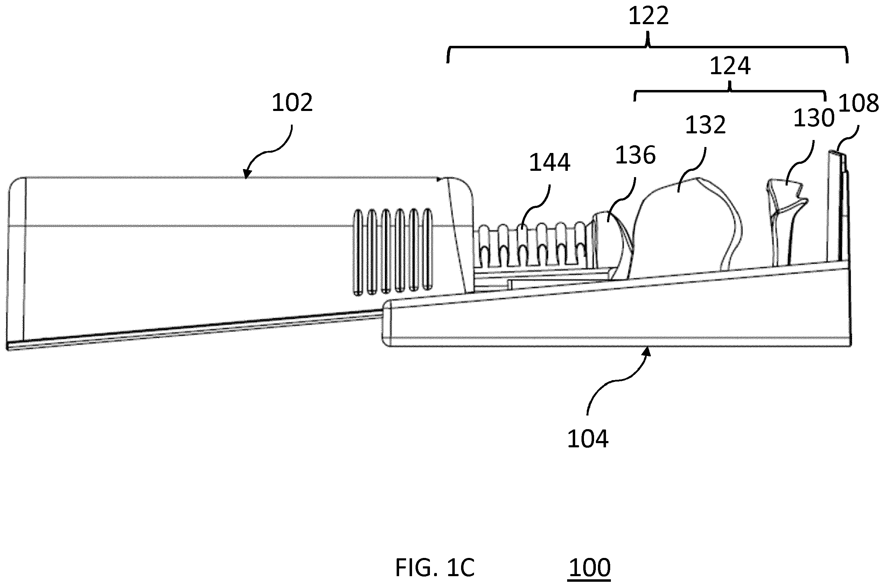

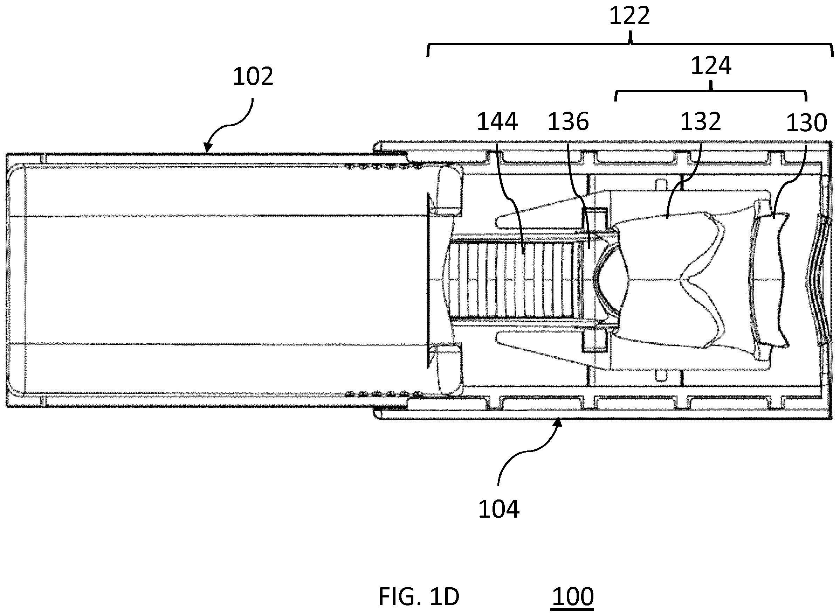

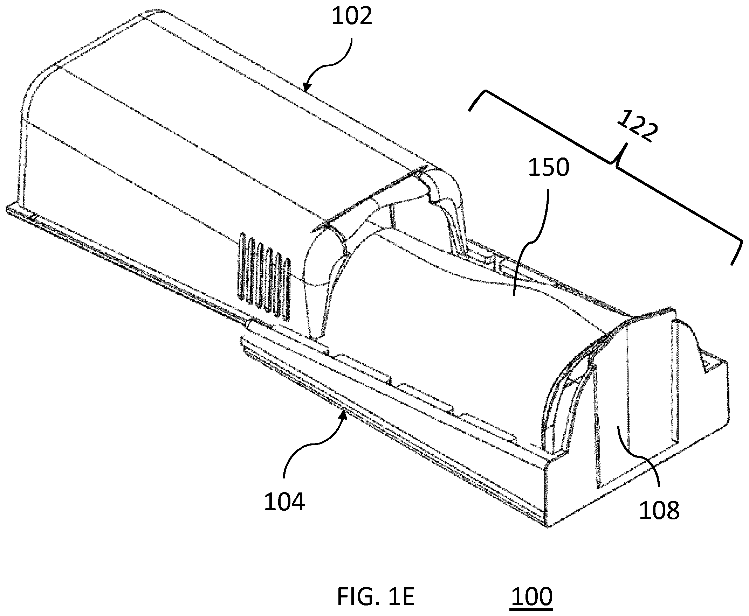

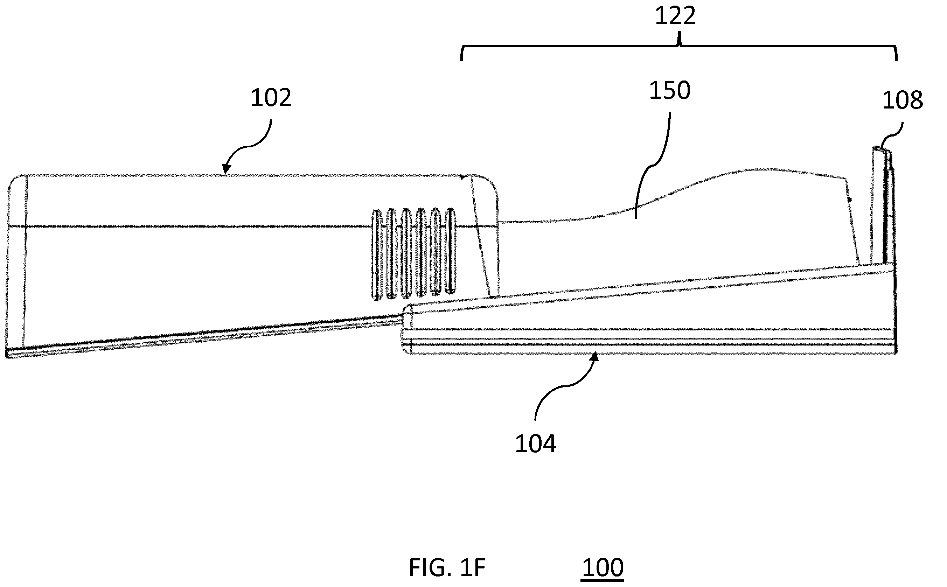

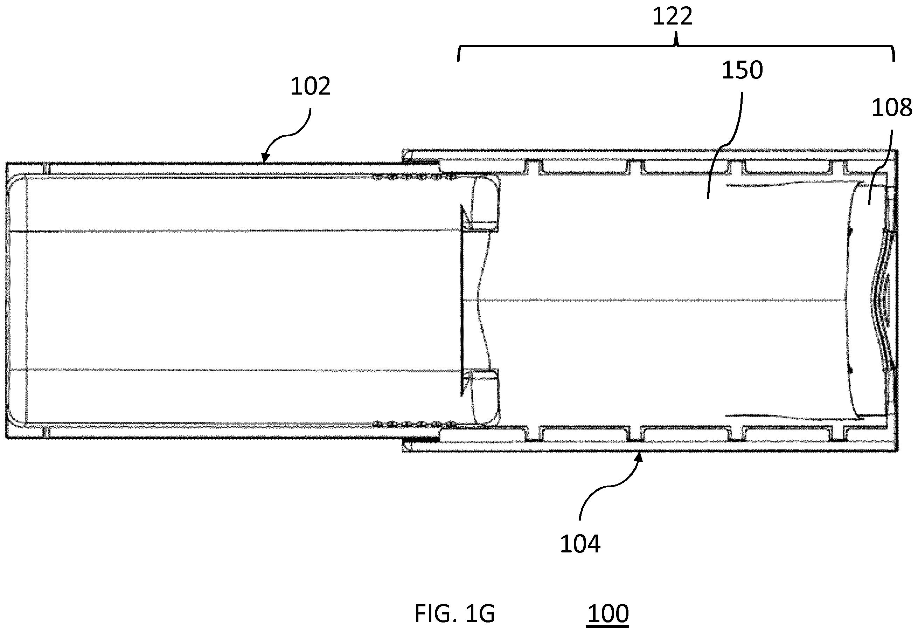

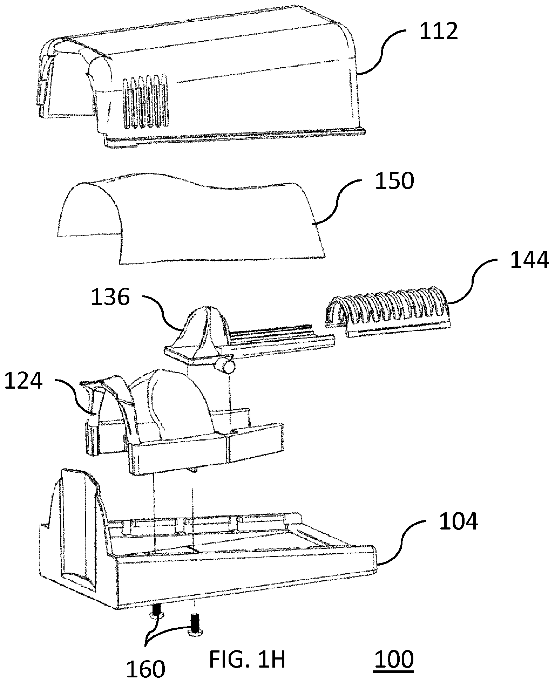

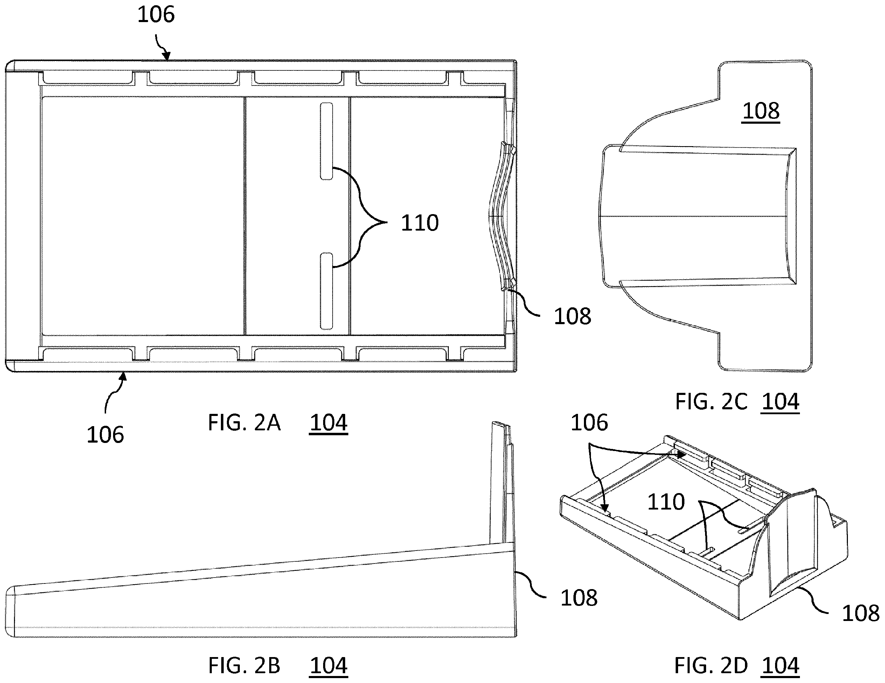

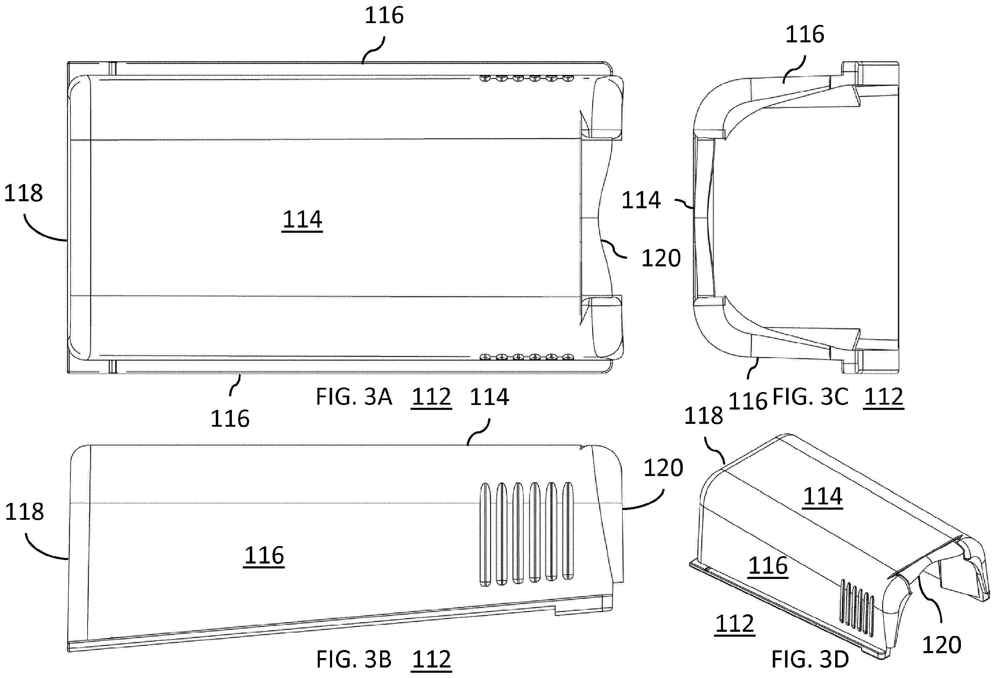

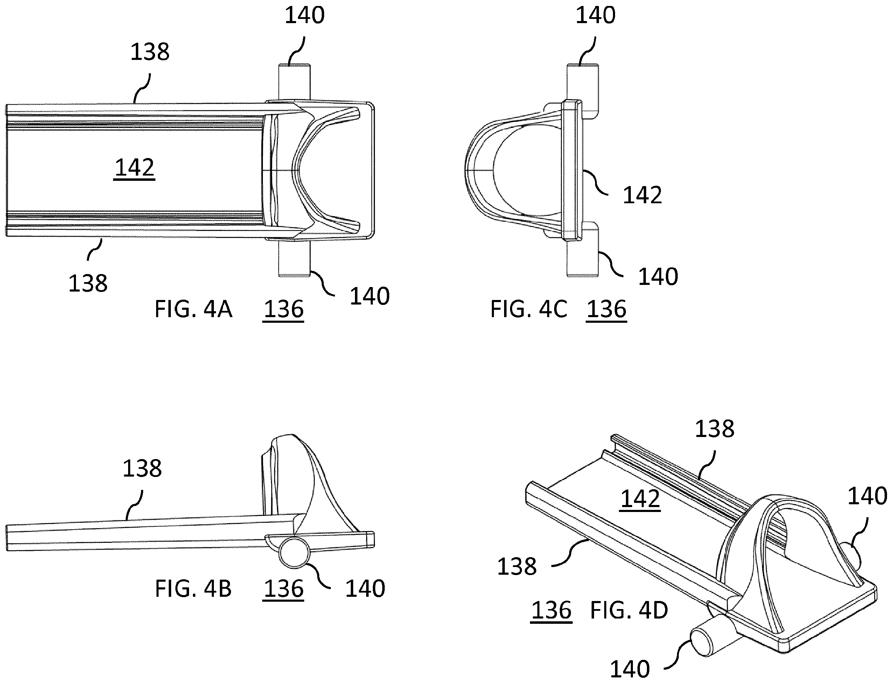

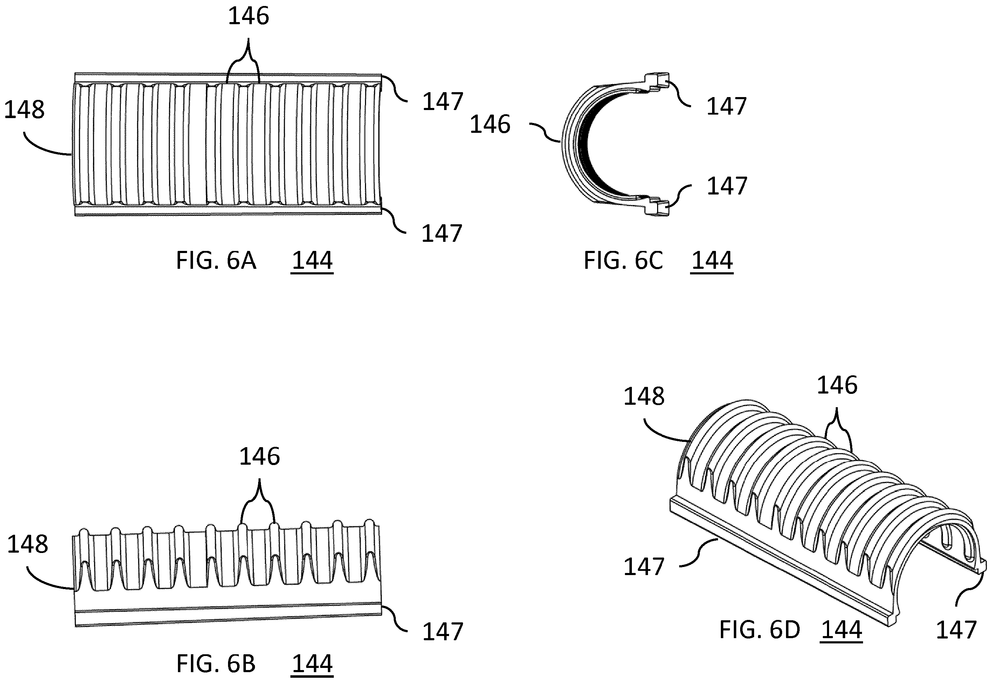

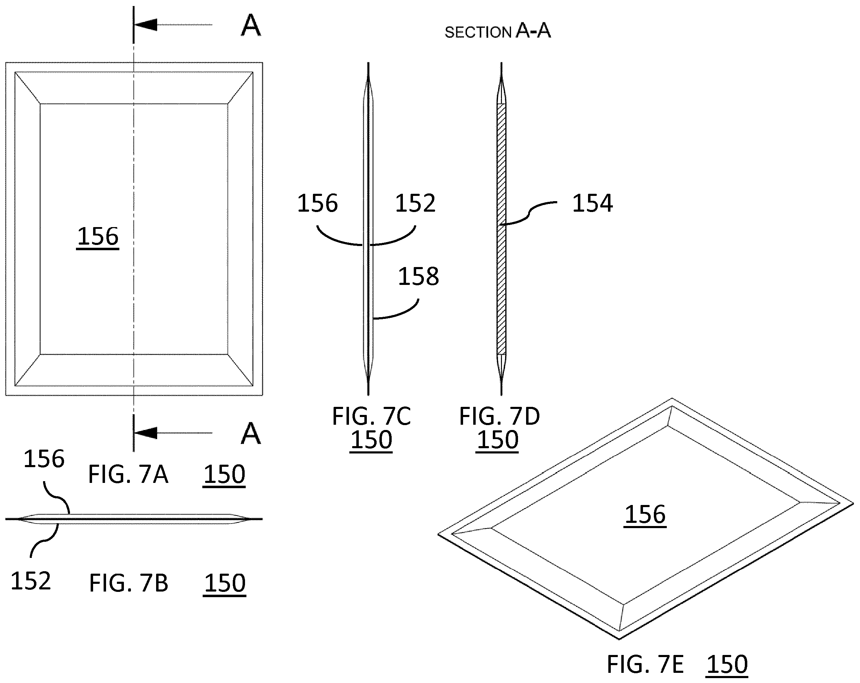

FIGs. 1A-7E illustrate a cricothyrotomy task trainer 100 according to certain embodiments of the disclosure. Trainer 100 includes (i) a case 102 having a base 104 and a cover 112 and (ii) a model 122 of a patient. The model 122, which is mounted within the base 104, includes a combined hyoid/thyroid analogue 124 that has an analogue 130 of the patient's hyoid bone and an analogue 132 of the patient's thyroid cartilage), a cricoid analogue 136 (i.e., an analogue of the patient's cricoid cartilage), a tracheal analogue 144 (i.e., an analogue of the patient's trachea), and a three-layer skin analogue 150 (i.e., an analogue of the patient's skin). The hyoid, thyroid, and cricoid analogues 130, 132, and 136 are referred to collectively as the laryngeal analogues.

The integral protective case 102 has a base 104 and a cover 112 that slides open to expose the model 122 for use. The sliding cover 112 includes top 114, lateral sides 116, and distal (inferior) end 118, which constitute additional elements of the model 122.

The proximal end 108 of the base 104 is flat, rises above the height of the hyoid/ thyroid, cricoid, and tracheal analogues 124, 136, and 144. The base 104 includes a sliding mechanism 106 for the cover 112. The proximal end 108 of the base 104 is intended to represent the position of the patient's chin above the laryngeal structures in the patient's neck.

The proximal end 120 of the cover 112 is another element of the model 122 that functions as an analogue of the patient's sternum and provides a sternal rest for the operator. When performing a cricothyrotomy, a critical component of proper practice is for the operator to rest their dominant hand holding the scalpel on the patient's sternum (for stabilization).

Two opposing, lateral struts 126 run along the base of the hyoid/thyroid analogue 124 extending from the thyroid analogue 132 (i) cranially toward the hyoid analogue 130 and (ii) caudally to an articulating connection 140 of the cricoid analogue 136 at notches 128.

The cricoid analogue 136 has a flat extension 142, which serves as an analogue of the flat posterior portion of the patient's trachea.

Cricothyrotomy trainers of the present disclosure can have anatomically correct laryngeal cartilages in both male and female forms and have lateral mobility approximating the side-to-side mobility of the human larynx (e.g., about 1-2 inches), even when the model 122 has an attached skin analogue 150 overlaying the laryngeal and tracheal analogues 130/132/136/144. Stabilizing the larynx from side-to-side movement (by the operator using their non-dominant hand) is a critical component of performing the cricothyrotomy procedure.

In certain embodiments, the lateral struts 126 have screws or bolts 160 that pass through horizontal, transverse slots 110 in the base 104. The enlarged heads of the screws or bolts 160 slide in the slots 110, allowing the side-to-side movement of the hyoid/thyroid analogue 124 (and thereby the side-to-side movement of the cricoid and tracheal analogues 136 and 144).

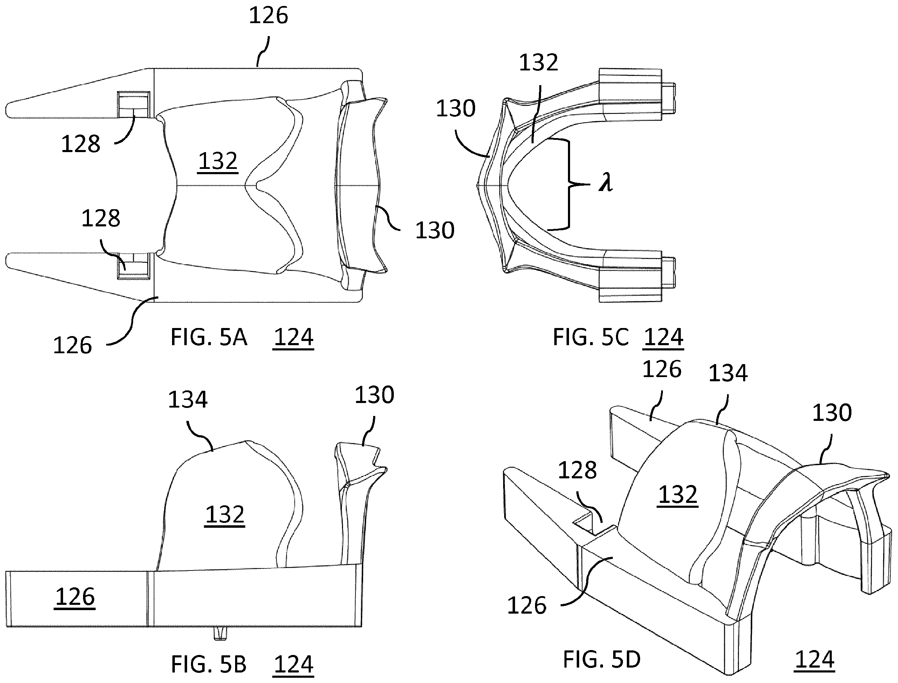

Male and female forms of the trainer 100 may have different angles λ (FIG. 5C) to the thyroid lamina 134 (e.g., approximately 90 degrees in the male form and approximately 120 degrees in the female form) and smaller overall dimensions (by approximately 15%) for the laryngeal and tracheal analogues 130/132/136/144 in the female form than in the male form.

The adhesive, three-layer skin analogue 150 attaches to the laryngeal analogues 130/132/136. The skin analogue 150 has an adhesive base layer 152 that represents the cricothyroid membrane, a middle layer 154 that represents subcutaneous tissue--which can be made either thin or thick using absorbent material to create a wet model, for representing bleeding, and a thin top layer 156 with a soft feel approximating human skin. When attached to the laryngeal analogues 130/132/136, the skin analogue 150 does not inhibit side-to-side movement of the laryngeal and tracheal analogues 130/132/136/144.

A relatively hard durometer plastic is used to form the hyoid/thyroid and cricoid analogues 124 and 136 as well as the base 104 and cover 112, while a softer durometer plastic is used for the tracheal analogue 144, which is externally, internally, and cross-sectionally anatomically representative of a human trachea, including having tracheal rings 146 that are palpable externally and internally. The flat posterior section 142 of the cricoid analogue 136 is representative of the membranous trachea. The tracheal rings 146 have an incomplete horseshoe shape with the rings present anteriorly, but not present on the flat posterior section 142 of the cricoid analogue 136.

The three-layer skin analogue 150 may be made from a thin rectangular plastic pouch approximately 4" by 6" of either 2 or 3 mil thickness having a side opening that allows insertion of the absorbent material as the middle layer 154, where the two layers of the pouch function as the base and top layers 152 and 156 of the skin analogue 150. The middle layer 154 may comprise either white or light beige cotton or Dacron that is loosely woven, allowing it to be cut to proper length, but also to break apart with the insertion of surgical instruments or a finger, as in the actual cricothyrotomy procedure. The skin analogue 150 may have an opaque covering either printed in a skin tone overlying the plastic material or made of an opaque skin-tone-colored plastic, such that, when the top layer is cut, the operator can distinguish the incision of the top layer 156 from the middle layer 154, and, if fluid (artificial blood) has been placed into the pouch, then the fluid will leak out, simulating bleeding. A backing 158 on the three-layer skin analogue 150 may be removed to expose the adhesive base layer 152. The base layer 152 or the backing 158 may have a drawing of the procedural landmarks, including the hyoid, thyroid, cricoid, trachea, and sternum.

The soft tracheal analogue 144 attaches to the harder laryngeal analogues 130/132/136 with the tracheal analogue 144 tightly abutting the more-rigid cricoid analogue 136 with a friction fit that allows the tracheal analogue 144 to be removed and replaced as needed should the tracheal analogue 144 be damaged or cut during training. In some embodiments, the cricoid analogue 136 has a flat base 142 that forms a longitudinal extension of the cricoid analogue 136. Alongside this flat base 142, the cricoid analogue 136 has two lateral ridges 138 that receive the two lateral rails 147 of the tracheal analogue 144 and between which the posterior section 148 of the softer durometer tracheal analogue 144 slides into place. The soft durometer plastic of the tracheal analogue 144 and the mechanical interaction between the cricoid and tracheal analogues 136 and 144 create enough friction so that the tracheal analogue 144 does not move out of position when performing the cricothyrotomy procedure on the model 122.

When a tube is inappropriately inserted between the hyoid bone analogue 130 and the thyroid cartilage analogue 132 of the unitary hyoid/thyroid analogue 124 at too steep an angle, the tube will pass below the cricoid cartilage analogue 136, thereby simulating the undesirable, real-life situation of the tube entering the esophagus posteriorly.

Although not shown in the figures, the bottom of the trainer 100 may have rubber feet that inhibit the trainer 100 from sliding on a flat surface.

### List of Figure Elements

| | |
|---|---|
| • 100: | cricothyrotomy task trainer |
| • 102: | case |
| • 104: | base |
| • 106: | sliding mechanism of base 104 |
| • 108: | proximal end of base 104 |
| • 110: | horizontal slots in base 104 |
| • 112: | cover |
| • 114: | top of cover 112 |
| • 116: | lateral sides of cover 112 |
| • 118: | distal (inferior) end of cover 112 |
| • 120: | proximal end of cover 112 |
| • 122: | model of a patient |
| • 124: | hyoid/thyroid analogue |
| • 126: | lateral struts of hyoid/thyroid analogue 124 |
| • 128: | notches of hyoid/thyroid analogue 124 |
| • 130: | hyoid bone analogue |
| • 132: | thyroid cartilage analogue |
| • 134: | thyroid lamina |
| • 136: | cricoid cartilage analogue |
| • 138: | ridges of cricoid analogue 136 |
| • 140: | articulating connection of cricoid analogue 136 |
| • 142: | flat extension of cricoid analogue 136 |
| • 144: | tracheal analogue |
| • 146: | tracheal rings |
| • 147: | lateral rails of tracheal analogue 144 |
| • 148: | posterior section of tracheal analogue 144 |
| • 150: | skin analogue |
| • 152: | base layer of skin analogue 150 |
| • 154: | middle layer of skin analogue 150 |
| • 156: | top layer of skin analogue 150 |
| • 158: | removable backing on skin analogue 150 |
| • 160: | screws/bolts |

In certain embodiments, the present disclosure is a trainer (e.g., 100) for cricothyrotomy. The trainer comprises a case (e.g., 102) and a model (e.g., 122) of a patient mounted within the case. The model comprises a hyoid analogue (e.g., 130); a thyroid analogue (e.g., 132) adjacent to the hyoid analogue; a cricoid analogue (e.g., 136) adjacent to the thyroid analogue; a tracheal analogue (e.g., 144) adjacent to the cricoid analogue; and a skin analogue (e.g., 150) adapted to cover the hyoid, thyroid, cricoid, and tracheal analogues.

In at least some of the above embodiments, the case comprises a base (e.g., 104) and a cover (e.g., 112), wherein the base and the cover are slidably interconnected to enable the cover to slide relative to the base to expose the model.

In at least some of the above embodiments, a proximal end (e.g., 108) of the base rises above the hyoid, thyroid, cricoid, and trachea analogues to represent the patient's chin.

In at least some of the above embodiments, after the cover has been slid relative to the base to expose the model, the cover represents the patient's sternum.

In at least some of the above embodiments, the hyoid, thyroid, and cricoid analogues have lateral mobility within the base representing side-to-side mobility of the patient's larynx.

In at least some of the above embodiments, the hyoid, thyroid, and cricoid analogues have the lateral mobility within the base with the skin analogue applied to the hyoid, thyroid, and cricoid analogues.

In at least some of the above embodiments, the base has at least one transverse slot (e.g., 110) that engages with at least one structure (e.g., 160) of the model to enable the lateral mobility.

In at least some of the above embodiments, the hyoid and thyroid analogues are part of a unitary hyoid/thyroid analogue structure (e.g., 124).

In at least some of the above embodiments, the unitary hyoid/thyroid analogue structure further comprises two lateral struts (e.g., 126) that support the hyoid and thyroid analogues.

In at least some of the above embodiments, each lateral strut has a notch (e.g., 128) that engages a different articulating connection (e.g., 140) of the cricoid analogue to connect the cricoid analogue to the unitary hyoid/thyroid analogue structure.

In at least some of the above embodiments, (i) the cricoid analogue comprises an extension (e.g., 142) having lateral ridges (e.g., 138) and (ii) the tracheal analogue comprises lateral rails (e.g., 147) that engage the lateral ridges to connect the tracheal analogue to the cricoid analogue.

In at least some of the above embodiments, the extension of the cricoid analogue has a flat surface to represent the patient's tracheal posterior surface.

In at least some of the above embodiments, the tracheal analogue has (i) a horseshoe-shaped cross section and (ii) horseshoe-shaped tracheal rings (e.g., 146) on the tracheal analogue's anterior surface.

In at least some of the above embodiments, the skin analogue comprises a middle layer (e.g., 154) between a base layer (e.g., 152) and a top layer (e.g., 156).

In at least some of the above embodiments, the base layer has an adhesive surface that enables the skin analogue to be removably applied to the hyoid, thyroid, and cricoid analogues.

In at least some of the above embodiments, the skin analogue further comprises a removable backing (e.g., 158) covering the adhesive surface of the base layer, such that, after removing the backing from the skin analogue and exposing the adhesive surface, the skin analogue is removably appliable to the hyoid, thyroid, and cricoid analogues.

In at least some of the above embodiments, the base layer represents the patient's cricothyroid membrane; the middle layer represents the patient's subcutaneous tissue; and the top layer represents the patient's skin.

In at least some of the above embodiments, the base and top layers are made of plastic; and the middle layer comprises a wet, absorbent material to represent bleeding by the patient.

In at least some of the above embodiments, the thyroid analogue's lamina (e.g., 134) forms an angle of approximately 90 degrees to represent a male patient.

In at least some of the above embodiments, the thyroid analogue's lamina (e.g., 134) forms an angle of approximately 120 degrees to represent a female patient.

In at least some of the above embodiments, the cricoid analogue is made of a relatively hard durometer material; and the tracheal analogue is made of a relatively soft durometer material that forms a removable, friction fit between the tracheal analogue and the cricoid analogue that enables the tracheal analogue to be replaceable.

Unless explicitly stated otherwise, each numerical value and range should be interpreted as being approximate as if the word "about" or "approximately" preceded the value or range.

The use of figure numbers and/or figure reference labels in the claims is intended to identify one or more possible embodiments of the claimed subject matter in order to facilitate the interpretation of the claims. Such use is not to be construed as necessarily limiting the scope of those claims to the embodiments shown in the corresponding figures.

Although the elements in the following method claims, if any, are recited in a particular sequence with corresponding labeling, unless the claim recitations otherwise imply a particular sequence for implementing some or all of those elements, those elements are not necessarily intended to be limited to being implemented in that particular sequence. Likewise, additional steps may be included in such methods, and certain steps may be omitted or combined, in methods consistent with various embodiments of the disclosure.

Reference herein to "one embodiment" or "an embodiment" means that a particular feature, structure, or characteristic described in connection with the embodiment can be included in at least one embodiment of the disclosure. The appearances of the phrase "in one embodiment" in various places in the specification are not necessarily all referring to the same embodiment, nor are separate or alternative embodiments necessarily mutually exclusive of other embodiments. The same applies to the term "implementation."

Unless otherwise specified herein, the use of the ordinal adjectives "first," "second," "third," etc., to refer to an object of a plurality of like objects merely indicates that different instances of such like objects are being referred to, and is not intended to imply that the like objects so referred-to have to be in a corresponding order or sequence, either temporally, spatially, in ranking, or in any other manner.

Also for purposes of this description, the terms "couple," "coupling," "coupled," "connect," "connecting," or "connected" refer to any manner known in the art or later developed in which energy is allowed to be transferred between two or more elements, and the interposition of one or more additional elements is contemplated, although not required. Conversely, the terms "directly coupled," "directly connected," etc., imply the absence of such additional elements. The same type of distinction applies to the use of terms "attached" and "directly attached," as applied to a description of a physical structure. For example, a relatively thin layer of adhesive or other suitable binder can be used to implement such "direct attachment" of the two corresponding components in such physical structure.

The described embodiments are to be considered in all respects as only illustrative and not restrictive. In particular, the scope of the disclosure is indicated by the appended claims rather than by the description and figures herein. All changes that come within the meaning and range of equivalency of the claims are to be embraced within their scope.

In this specification including any claims, the term "each" may be used to refer to one or more specified characteristics of a plurality of previously recited elements or steps. When used with the open-ended term "comprising," the recitation of the term "each" does not exclude additional, unrecited elements or steps. Thus, it will be understood that an apparatus may have additional, unrecited elements and a method may have additional, unrecited steps, where the additional, unrecited elements or steps do not have the one or more specified characteristics.

As used herein, "at least one of the following: <a list of two or more elements>" and "at least one of <a list of two or more elements>" and similar wording, where the list of two or more elements are joined by "and" or "or", mean at least any one of the elements, or at least any two or more of the elements, or at least all the elements. For example, the phrases "at least one of A and B" and "at least one of A or B" are both to be interpreted to have the same meaning, encompassing the following three possibilities: 1- only A; 2- only B; 3- both A and B.

The embodiments covered by the claims in this application are limited to embodiments that (1) are enabled by this specification and (2) correspond to statutory subject matter. Non-enabled embodiments and embodiments that correspond to non-statutory subject matter are explicitly disclaimed even if they fall within the scope of the claims.

While preferred embodiments of the disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the technology of the disclosure. It is intended that the following claims define the scope of the invention and that methods and structures within the scope of these claims and their equivalents be covered thereby.

## Claims

1. A trainer (100) for cricothyrotomy, the trainer comprising:
a case (102); and
a model (122) of a patient mounted within the case, the model comprising:
a hyoid analogue (130);
a thyroid analogue (132) adjacent to the hyoid analogue;
a cricoid analogue (136) adjacent to the thyroid analogue;
a tracheal analogue (144) adjacent to the cricoid analogue; and
a skin analogue (150) adapted to cover the hyoid, thyroid, cricoid, and tracheal analogues **CHARACTERISED IN THAT**:
the case comprises a base (104) and a cover (112), wherein the base and the cover are slidably interconnected to enable the cover to slide relative to the base to expose the model; and
the hyoid, thyroid, and cricoid analogues have lateral mobility within the base representing side-to-side mobility of the patient's larynx with or without the skin analogue applied to the hyoid, thyroid, and cricoid analogues.

2. The trainer of claim 1, wherein a proximal end (108) of the base rises above the hyoid, thyroid, cricoid, and trachea analogues to represent the patient's chin.

3. The trainer of any of claims 1-2, wherein, after the cover has been slid relative to the base to expose the model, the cover represents the patient's sternum.

4. The trainer of any of claims 1-3, wherein the base has at least one transverse slot (110) that engages with at least one structure (160) of the model to enable the lateral mobility.

5. The trainer of any of claims 1-4, wherein the hyoid and thyroid analogues are part of a unitary hyoid/thyroid analogue structure (124).

6. The trainer of claim 5, wherein the unitary hyoid/thyroid analogue structure further comprises two lateral struts (126) that support the hyoid and thyroid analogues.

7. The trainer of claim 6, wherein each lateral strut has a notch (128) that engages a different articulating connection (140) of the cricoid analogue to connect the cricoid analogue to the unitary hyoid/thyroid analogue structure.

8. The trainer of any of claims 1-7, wherein:
the cricoid analogue comprises an extension (142) having lateral ridges (138); and
the tracheal analogue comprises lateral rails (147) that engage the lateral ridges to connect the tracheal analogue to the cricoid analogue.

9. The trainer of claim 8, wherein:
the extension of the cricoid analogue has a flat surface to represent the patient's tracheal posterior surface; and
the tracheal analogue has (i) a horseshoe-shaped cross section and (ii) horseshoe-shaped tracheal rings (146) on the tracheal analogue's anterior surface.

10. The trainer of any of claims 1-9, wherein:
the skin analogue comprises a middle layer (154) between a base layer (152) and a top layer (156);
the base layer has an adhesive surface that enables the skin analogue to be removably applied to the hyoid, thyroid, and cricoid analogues; and
the skin analogue further comprises a removable backing (158) covering the adhesive surface of the base layer, such that, after removing the backing from the skin analogue and exposing the adhesive surface, the skin analogue is removably appliable to the hyoid, thyroid, and cricoid analogues.

11. The trainer of claim 10, wherein:
the base layer represents the patient's cricothyroid membrane;
the middle layer represents the patient's subcutaneous tissue; and
the top layer represents the patient's skin.

12. The trainer of claim 11, wherein:
the base and top layers are made of plastic; and
the middle layer comprises a wet, absorbent material to represent bleeding by the patient.

13. The trainer of any of claims 1-12, wherein:
the cricoid analogue is made of a relatively hard durometer material; and
the tracheal analogue is made of a relatively soft durometer material that forms a removable, friction fit between the tracheal analogue and the cricoid analogue that enables the tracheal analogue to be replaceable.

## Patentansprüche

1. Trainingsvorrichtung (100) für Koniotomie, wobei die Trainingsvorrichtung aufweist:
ein Gehäuse (102) und
ein Modell (122) eines Patienten, das in dem Gehäuse angebracht ist, wobei das Modell aufweist:
eine Zungenbein-Nachbildung (130),
eine Schilddrüsen-Nachbildung (132), die zu der Zungenbein-Nachbildung benachbart ist,
eine Ringknorpel-Nachbildung (136), die zu der Schilddrüsen-Nachbildung benachbart ist,
eine Luftröhren-Nachbildung (144), die zu der Ringknorpel-Nachbildung benachbart ist, und
eine Hautnachbildung (150), die angepasst ist, um die Zungenbein-, Schilddrüsen-, Ringknorpel- und Luftröhren-Nachbildungen zu bedecken, **DADURCH GEKENNZEICHNET, DASS**
das Gehäuse eine Basis (104) und eine Abdeckung (112) aufweist, wobei die Basis und die Abdeckung verschiebbar miteinander verbunden sind, um es der Abdeckung zu ermöglichen, relativ zur Basis verschoben zu werden, um das Modell freizulegen, und
die Zungenbein-, Schilddrüsen- und Ringknorpel-Nachbildungen eine seitliche Beweglichkeit innerhalb der Basis haben, die die seitliche Beweglichkeit des Kehlkopfs des Patienten mit oder ohne die auf die Zungenbein-, Schilddrüsen- und Ringknorpel-Nachbildungen aufgebrachte Haut-Nachbildung darstellt.

2. Trainingsvorrichtung gemäß Anspruch 1, wobei ein proximales Ende (108) der Basis über die Zungenbein-, Schilddrüsen-, Ringknorpel- und Luftröhren-Nachbildungen hinausragt, um das Kinn des Patienten darzustellen.

3. Trainingsvorrichtung gemäß irgendeinem der Ansprüche 1-2, wobei die Abdeckung, nachdem die Abdeckung relativ zur Basis verschoben worden ist, um das Modell freizulegen, das Brustbein des Patienten darstellt.

4. Trainingsvorrichtung gemäß irgendeinem der Ansprüche 1-3, wobei die Basis mindestens einen Querschlitz (110) hat, der mit mindestens einer Struktur (160) des Modells im Eingriff steht, um die seitliche Beweglichkeit zu ermöglichen.

5. Trainingsvorrichtung gemäß irgendeinem der Ansprüche 1-4, wobei die Zungenbein- und Schilddrüsen-Nachbildungen Teil einer einheitlichen Zungenbein/Schilddrüsen-Nachbildungsstruktur (124) sind.

6. Trainingsvorrichtung gemäß Anspruch 5, wobei die einheitliche Zungenbein/Schilddrüsen- Nachbildungsstruktur ferner zwei seitliche Holme (126) aufweist, die die Zungenbein- und Schilddrüsen-Nachbildungen stützen.

7. Trainingsvorrichtung gemäß Anspruch 6, wobei jeder seitliche Holm eine Aussparung (128) hat, die mit einer anderen Gelenkverbindung (140) der Ringknorpel-Nachbildung im Eingriff ist, um die Ringknorpel-Nachbildung mit der einheitlichen Zungenbein/Schilddrüsen-Nachbildungsstruktur zu verbinden.

8. Trainingsvorrichtung gemäß irgendeinem der Ansprüche 1-7, wobei:
die Ringknorpel-Nachbildung eine Verlängerung (142) mit seitlichen Rippen (138) aufweist, und
die Luftröhren-Nachbildung seitliche Schienen (147) aufweist, die mit den seitlichen Rippen im Eingriff sind, um
die Luftröhren-Nachbildung mit der Ringknorpel-Nachbildung zu verbinden.

9. Trainingsvorrichtung gemäß Anspruch 8, wobei:
die Verlängerung der Ringknorpel-Nachbildung eine flache Fläche aufweist, um die hintere Luftröhrenfläche des Patienten darzustellen, und
die Luftröhren-Nachbildung (i) einen hufeisenförmigen Querschnitt und (ii) hufeisenförmige Luftröhrenringe (146) auf der vorderen Fläche der Luftröhren-Nachbildung aufweist.

10. Trainingsvorrichtung gemäß irgendeinem der Ansprüche 1-9, wobei:
die Haut-Nachbildung eine mittlere Schicht (154) zwischen einer Basisschicht (152) und einer oberen Schicht (156) aufweist,
die Basisschicht eine Klebefläche hat, die es ermöglicht, die Haut-Nachbildung abnehmbar auf die Zungenbein-, Schilddrüsen- und Ringknorpel-Nachbildungen aufzubringen, und
die Haut-Nachbildung ferner eine entfernbare Unterlage (158) aufweist, die die Klebefläche der Basisschicht bedeckt, so dass die Haut-Nachbildung nach dem Entfernen der Unterlage von der Haut-Nachbildung und dem Freilegen der Klebefläche entfernbar auf die Zungenbein-, Schilddrüsen- und Ringknorpel-Nachbildungen aufgebracht werden kann.

11. Trainingsvorrichtung gemäß Anspruch 10, wobei:
die Basisschicht die Kehlkopf-Membran des Patienten darstellt,
die mittlere Schicht das subkutane Gewebe des Patienten darstellt und
die obere Schicht die Haut des Patienten darstellt.

12. Trainingsvorrichtung gemäß Anspruch 11, wobei:
die Basis- und die obere Schicht aus Kunststoff gemacht sind und
die mittlere Schicht ein feuchtes, absorbierendes Material aufweist, um Blutungen des Patienten darzustellen.

13. Trainingsvorrichtung gemäß irgendeinem der Ansprüche 1-12, wobei:
die Ringknorpel-Nachbildung aus einem relativ-harten-Durometer-Material gemacht ist und
die Luftröhren-Nachbildung aus einem relativ-weichen-Durometer-Material gemacht ist, das eine entfernbaren Reibungssitz zwischen der Luftröhren-Nachbildung und der Ringknorpel-Nachbildung bildet, welcher es der Luftröhren-Nachbildung ermöglicht, ausgetauscht zu werden.

## Revendications

1. Simulateur d'entraînement (100) de cricothyrotomie, le simulateur d'entraînement comprenant :
un boîtier (102) ; et
un modèle (122) d'un patient monté à l'intérieur du boîtier, le modèle comprenant :
un analogue hyoïde (130) ;
un analogue thyroïdien (132) adjacent à l'analogue hyoïde ;
un analogue cricoïde (136) adjacent à l'analogue thyroïdien ;
un analogue trachéal (144) adjacent à l'analogue cricoïde ; et
un analogue cutané (150) conçu pour recouvrir les analogues hyoïde, thyroïdien, cricoïde et trachéal **CARACTÉRISÉ EN CE QUE** :
le boîtier comprend une base (104) et un couvercle (112), dans lequel la base et le couvercle sont raccordés entre eux de manière coulissante pour permettre au couvercle de coulisser par rapport à la base pour exposer le modèle ; et
les analogues hyoïde, thyroïdien et cricoïde présentent une mobilité latérale à l'intérieur de la base représentant la mobilité côte à côte du larynx du patient avec ou sans l'analogue cutané appliqué aux analogues hyoïde, thyroïdien et cricoïde.

2. Simulateur d'entraînement selon la revendication 1, dans lequel une extrémité proximale (108) de la base s'élève au-dessus des analogues hyoïde, thyroïdien, cricoïde et trachéal pour représenter le menton du patient.

3. Simulateur d'entraînement selon l'une quelconque des revendications 1 à 2, dans lequel, après que le couvercle a été coulissé par rapport à la base pour exposer le modèle, le couvercle représente le sternum du patient.

4. Simulateur d'entraînement selon l'une quelconque des revendications 1 à 3, dans lequel la base présente au moins une fente transversale (110) qui vient en prise avec au moins une structure (160) du modèle pour permettre la mobilité latérale.

5. Simulateur d'entraînement selon l'une quelconque des revendications 1 à 4, dans lequel les analogues hyoïde et thyroïdien font partie d'une structure d'analogue hyoïde/thyroïdien unitaire (124).

6. Simulateur d'entraînement selon la revendication 5, dans lequel la structure d'analogue hyoïde/thyroïdien unitaire comprend en outre deux entretoises latérales (126) qui supportent les analogues hyoïde et thyroïdien.

7. Simulateur d'entraînement selon la revendication 6, dans lequel chaque entretoise latérale présente une encoche (128) qui vient en prise dans un raccord d'articulation différent (140) de l'analogue cricoïde pour raccorder l'analogue cricoïde à la structure d'analogue hyoïde/thyroïdien unitaire.

8. Simulateur d'entraînement selon l'une quelconque des revendications 1 à 7, dans lequel :
l'analogue cricoïde comprend une extension (142) présentant des arêtes latérales (138) ; et
l'analogue trachéal comprend des rails latéraux (147) qui viennent en prise dans les arêtes latérales pour raccorder l'analogue trachéal à l'analogue cricoïde.

9. Simulateur d'entraînement selon la revendication 8, dans lequel :
l'extension de l'analogue cricoïde présente une surface plane pour représenter la surface postérieure trachéale du patient ; et
l'analogue trachéal présente (i) une section transversale en forme de fer à cheval et (ii) des anneaux trachéaux en forme de fer à cheval (146) sur la surface antérieure de l'analogue trachéal.

10. Simulateur d'entraînement selon l'une quelconque des revendications 1 à 9, dans lequel :
l'analogue cutané comprend une couche intermédiaire (154) entre une couche de base (152) et une couche supérieure (156) ;
la couche de base présente une surface adhésive qui permet à l'analogue cutané d'être appliqué de manière amovible sur les analogues hyoïde, thyroïdien et cricoïde ; et
l'analogue cutané comprend en outre un support amovible (158) recouvrant la surface adhésive de la couche de base, de sorte qu'après avoir retiré le support de l'analogue cutané et exposé la surface adhésive, l'analogue cutané soit applicable de manière amovible aux analogues hyoïde, thyroïdien et cricoïde.

11. Simulateur d'entraînement selon la revendication 10, dans lequel :
la couche de base représente la membrane cricothyroïdienne du patient ;
la couche intermédiaire représente le tissu sous-cutané du patient ; et
la couche supérieure représente la peau du patient.

12. Simulateur d'entraînement selon la revendication 11, dans lequel :
les couches de base et supérieure sont composées de plastique ; et
la couche intermédiaire comprend un matériau absorbant humide pour représenter le saignement par le patient.

13. Simulateur d'entraînement selon l'une quelconque des revendications 1 à 12, dans lequel :
l'analogue cricoïde est composé d'un matériau de duromètre relativement dur ; et
l'analogue trachéal est composé d'un matériau de duromètre relativement souple qui forme un ajustement par friction amovible entre l'analogue trachéal et l'analogue cricoïde qui permet de remplacer l'analogue trachéal.
